(19) Europäisches Patentamt
European Patent Office
Office européen des brevets

(11) **EP 3 423 180 B1**

(12) **FASCICULE DE BREVET EUROPEEN**

(45) Date de publication et mention
de la délivrance du brevet:
**08.10.2025  Bulletin 2025/41**

(21) Numéro de dépôt: **17707075.2**

(22) Date de dépôt: **01.03.2017**

(51) Classification Internationale des Brevets (IPC):
**B01F 29/00** *(2022.01)*     **B01F 29/62** *(2022.01)*

(52) Classification Coopérative des Brevets (CPC):
**B01F 29/40111; B01F 29/40112; B01F 29/40113;
B01F 29/4033; B01F 29/62**

(86) Numéro de dépôt international:
**PCT/EP2017/054813**

(87) Numéro de publication internationale:
**WO 2017/149034 (08.09.2017 Gazette 2017/36)**

(54) **MELANGEUR SANS PALE ET PROCÉDÉ**

FLÜGELLOSER MISCHER UND VERFAHREN

BLADELESS MIXER AND METHOD

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:   **01.03.2016   FR 1651703**

(43) Date de publication de la demande:
**09.01.2019   Bulletin 2019/02**

(73) Titulaires:
- **Centre National de la Recherche Scientifique
  CNRS
  75794 Paris Cedex 16 (FR)**
- **Université d'Aix Marseille
  13284 Marseille Cedex 07 (FR)**
- **Ecole Centrale de Marseille
  13013 Marseille (FR)**

(72) Inventeurs:
- **MEUNIER, Patrice
  13380 Plan de Cuques (FR)**
- **MANASSEH, Richard
  Melbourne
  Victoria 3183 (AU)**

(74) Mandataire: **Cabinet Camus Lebkiri
25 rue de Maubeuge
75009 Paris (FR)**

(56) Documents cités:
EP-A1- 1 374 991     EP-A1- 2 893 973
FR-A- 1 362 354      GB-A- 2 258 176
US-A- 4 435 082

EP 3 423 180 B1

**Description**

**DOMAINE TECHNIQUE DE L'INVENTION**

Le domaine technique de l'invention est celui des mélangeurs. La présente invention concerne un mélangeur sans pale ainsi qu'un procédé de mélange.

**ARRIERE-PLAN TECHNOLOGIQUE DE L'INVENTION**

Pour le mélange d'un liquide, l'utilisation d'un mélangeur comportant une pale en rotation est connue. La pale en rotation crée toutefois un fort cisaillement au sein du liquide, par décollement de couche limite. De plus, l'utilisation d'une pale nécessite généralement de nettoyer régulièrement ladite pale et rend difficile voire impossible de garantir que le liquide à mélanger ne sera pas pollué à cause de ladite pale.

Le problème du fort cisaillement est notamment connu dans le domaine des biotechnologies, et par exemple dans le domaine de la culture de cellules en solution dans un bioréacteur. Les cellules cultivées ont en effet besoin d'un apport régulier en oxygène et d'une évacuation régulière du dioxyde de carbone qu'elles rejettent. L'apport en oxygène et l'évacuation du dioxyde de carbone sont typiquement assurés par une pale en rotation. Mais le fort cisaillement créé par la pale en rotation peut tuer les cellules cultivées, qui préfèrent un milieu relativement immobile.

Afin de mélanger un liquide en s'affranchissant de l'utilisation d'une pale, il est connu d'injecter des bulles d'air en partie inférieure d'une cuve contenant le liquide à mélanger. Une telle solution présente notamment l'inconvénient de nécessiter un dispositif d'injection de bulles d'air. Il existe également des applications dans lesquelles l'injection de bulles d'air est contre-indiquée.

Des documents de l'art antérieur divulguent des techniques permettant le mélange d'un liquide sans pale. Le document FR 1 362 354 A divulgue un mélangeur et un procédé selon les préambules des revendications 1 et 6. Le document décrit notamment un mélangeur sans pale dans lequel l'effet de mélange est obtenu grâce à un fond profilé du récipient et à des déflecteurs. Le document EP 2 893 973 A1 décrit un mélangeur sans pale dans lequel l'effet de mélange est obtenu grâce à une source d'ondes ultrasonores.

Il est donc recherché un dispositif permettant de mélanger un liquide avec un faible taux de cisaillement au sein du liquide et un faible risque de pollution du liquide, tout en s'affranchissant de l'utilisation d'un dispositif complexe d'injection de bulles d'air, de déflecteurs ou d'ondes ultrasonores.

**RESUME DE L'INVENTION**

L'invention offre une solution aux problèmes évoqués précédemment en proposant un dispositif de mélange d'un liquide sans pale et sans injection de bulles d'air.

Un aspect de l'invention concerne un mélangeur sans pale pour le mélange d'un liquide selon la revendication 1.

Dans le présent document, on entend par « récipient cylindrique » un récipient ayant une paroi latérale définie par une droite appelée génératrice, passant par un point variable décrivant une courbe, appelée courbe directrice, et gardant une direction fixe. On entend par « récipient sensiblement cylindrique » un récipient cylindrique ou tronconique. En effet, les variations de diamètre de la section du récipient ne perturbent pas le fonctionnement du mélangeur dans la mesure où elles restent faibles. On conçoit que la fabrication du récipient peut conduire à des formes qui ne sont pas parfaitement cylindriques, notamment des formes tronconiques. On entend par liquide à mélanger une composition liquide comprenant un liquide et au moins une espèce à mélanger, à titre d'exemple, l'espèce à mélanger peut être un second liquide et/ou un scalaire passif n'influant pas sur les propriétés d'écoulement du liquide tel qu'un colorant ou un gaz dissout, tel que du $CO_2$ ou de l'$O_2$ dissout dans une solution aqueuse.

Dans le présent document, le « rayon R du récipient sensiblement cylindrique d'axe A » est la distance la plus petite entre l'axe A du récipient et une paroi latérale du récipient.

D'une manière générale, un mode propre inertiel peut être défini comme un mouvement global d'un liquide tournant, le mouvement étant :

- périodique ou stationnaire en temps,
- sinusoïdal selon l'angle azimutal θ, et
- forcé par une perturbation du liquide.

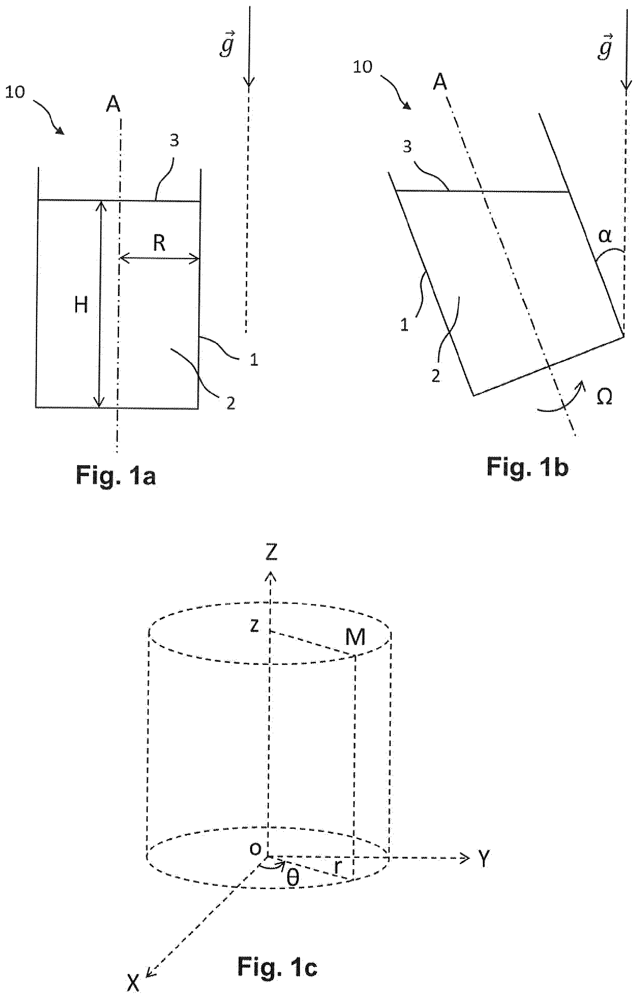

On entend par « angle azimutal θ » l'angle permettant de repérer la position d'un point en coordonnées cylindriques. La figure 1c montre schématiquement un point M de coordonnées cylindriques (r, θ, z).

On entend par « le mouvement est sinusoïdal selon l'angle azimutal θ » le fait que, en tout point du liquide non contraint par une paroi du récipient, chaque composante de la vitesse s'exprime en sin(mθ), avec m le nombre d'onde azimutal.

On force avantageusement les modes propres inertiels de nombre d'onde azimutal m = 1 en inclinant le récipient en rotation de manière que l'axe A forme l'angle $\alpha$ par rapport à la direction verticale.

On choisit le rapport d'aspect H/R de la hauteur H sur le rayon R de manière à se placer dans une résonance d'un desdits modes propres inertiels. De forts mouvements de grande échelle se produisent alors dans le volume du liquide en résonance, permettant un mélange efficace avec un taux de cisaillement très faible.

La vitesse angulaire de rotation $\Omega$ du récipient est choisie de manière à observer une résonance instable du mode propre inertiel du liquide placé dans le récipient incliné et en rotation. On entend par « instable » le fait que d'autres mouvements, différents du mouvement du mode propre inertiel, apparaissent au sein du liquide sans contrainte extérieure supplémentaire. Le mélange au sein du liquide est en effet meilleur lorsque la résonance est instable. La présence d'une résonance instable d'un mode propre inertiel dans un fluide se vérifie notamment en faisant apparaître les courants dynamiques au sein du fluide. Pour ce faire, on peut typiquement ajouter des paillettes de mica dans le liquide à mélanger puis illuminer ces paillettes au moyen d'une nappe laser verticale passant par l'axe du cylindre. On peut ainsi visualiser la structure spatiale de l'écoulement et donc vérifier la présence d'une résonance instable d'un mode propre inertiel. Une résonance instable d'un mode propre inertiel fait en effet apparaître des structures instationnaires caractéristiques, dont la luminosité oscille rapidement à proximité du bord du récipient. On entend par « structure instationnaire » une structure instable dans le temps.

Un autre aspect de l'invention concerne un procédé de mélange d'un liquide au moyen d'un mélangeur sans pale selon la revendication 6.

- Dans un premier mode de réalisation, le rapport d'aspect H/R de la hauteur H sur le rayon R est choisi tel que :

$$1{,}79 \times k \leq \frac{H}{R} \leq 2{,}19 \times k$$

k étant égal à 1.
- Selon le premier mode de réalisation et pour k = 1, la vitesse angulaire de rotation $\Omega$ est choisie telle que :

$$\frac{\Omega \times R^2 \times \alpha}{\nu} > 1000$$

pour laquelle la formule $\frac{\Omega \times R^2}{\nu}$ correspondant au nombre de Reynolds

selon un mode de réalisation selon l'invention :

$$\frac{\Omega \times R^2 \times \alpha}{\nu} > 3000 \quad \text{avec } \alpha \text{ inférieur ou égale à 2°}$$

selon un autre mode de réalisation selon l'invention :

$$\frac{\Omega \times R^2 \times \alpha}{\nu} > 5000 \quad \text{avec } \alpha \text{ compris entre 3° et 7°}$$

avec $\Omega$ la vitesse angulaire de rotation exprimée en rad/s, R le rayon du récipient exprimé en m, $\alpha$ l'angle d'inclinaison entre l'axe A et la direction verticale exprimée en degré ° et v la viscosité cinématique du liquide à mélanger exprimée en $m^2/s$. On favorise ainsi une résonance instable du premier mode propre inertiel. Selon un mode de réalisation de l'invention, $\Omega$ est constante.

- Dans un deuxième mode de réalisation, le rapport d'aspect H/R de la hauteur H sur le rayon R est choisi tel que :

$$0{,}86 \times k \leq \frac{H}{R} \leq 1{,}06 \times k$$

k étant égal à 1.

- Selon le deuxième mode de réalisation et pour k = 1, la vitesse angulaire de rotation Ω est choisie telle que :

$$\frac{\Omega \times R^2 \times \alpha}{\nu} > 15000$$ et α est compris entre 5° et 10°

selon un autre mode dé réalisation, $\frac{\Omega \times R^2 \times \alpha}{\nu} > 30000$ et α inférieur ou égal à 5° avec Ω la vitesse angulaire de rotation exprimée en rad/s, R le rayon du récipient exprimé en m, α l'angle d'inclinaison entre l'axe A et la direction verticale exprimée en degré ° et v la viscosité cinématique du liquide à mélanger exprimée en m$^2$/s. On favorise ainsi une résonance instable du deuxième mode propre inertiel. Selon un mode de réalisation de l'invention, Ω est constante.

- Dans un troisième mode de réalisation, le rapport d'aspect H/R de la hauteur H sur le rayon R est choisi tel que :

$$0,56 \times k \leq \frac{H}{R} \leq 0,68 \times k$$

k étant égal à 1.
- Selon le troisième mode de réalisation et pour k = 1, la vitesse angulaire de rotation Ω est choisie telle que :

$$\frac{\Omega \times R^2 \times \alpha}{\nu} > 50000$$

avec Ω la vitesse angulaire de rotation exprimée en rad/s, R le rayon du récipient exprimé en m, α l'angle d'inclinaison entre l'axe A et la direction verticale exprimée en degré ° et v la viscosité cinématique du liquide à mélanger exprimée en m$^2$/s. On favorise ainsi une résonance instable du troisième mode propre inertiel. Selon un mode de réalisation de l'invention, Ω est constante.
- Selon un mode de réalisation particulier de l'invention, une résonance instable d'un mode propre inertiel peut être favorisée en faisant varier la vitesse angulaire de rotation Ω, avec une variation de la vitesse angulaire de rotation Ω préférentiellement inférieure ou égale à 25% au cours d'une révolution complète du récipient.

L'invention et ses différentes applications seront mieux comprises à la lecture de la description qui suit et à l'examen des figures qui l'accompagnent.

**BREVE DESCRIPTION DES FIGURES**

Les figures sont présentées à titre indicatif et nullement limitatif de l'invention.

- La figure 1a montre schématiquement un mélangeur sans pale selon un mode de réalisation de l'invention dans une position de repos.
- La figure 1b montre schématiquement le mélangeur sans pale de la figure 1a dans une position de fonctionnement.
- La figure 1c montre schématiquement un système de coordonnées cylindriques.
- La figure 2a montre schématiquement une vue en coupe d'un premier récipient cylindrique d'un mélangeur sans pale selon un mode de réalisation de l'invention.
- La figure 2b montre schématiquement une vue en coupe d'un deuxième récipient cylindrique d'un mélangeur sans pale selon un mode de réalisation de l'invention.
- La figure 3a montre schématiquement une vue en coupe d'un troisième récipient cylindrique d'un mélangeur sans pale selon un mode de réalisation de l'invention.
- La figure 3b montre schématiquement une vue en coupe d'un quatrième récipient cylindrique d'un mélangeur sans pale selon un mode de réalisation de l'invention.
- La figure 4a montre schématiquement une première vue en perspective d'un récipient sensiblement cylindrique d'un mélangeur sans pale selon un mode de réalisation de l'invention.
- La figure 4b montre schématiquement une deuxième vue en perspective d'un récipient sensiblement cylindrique d'un mélangeur sans pale selon un mode de réalisation de l'invention.

- La figure 4c montre schématiquement une troisième vue en perspective d'un récipient sensiblement cylindrique d'un mélangeur sans pale selon un mode de réalisation de l'invention.

## DESCRIPTION DETAILLEE D'AU MOINS UN MODE DE REALISATION DE L'INVENTION

Sauf précision contraire, un même élément apparaissant sur des figures différentes présente une référence unique.

La figure 1a montre schématiquement un mélangeur 10 sans pale selon un mode de réalisation de l'invention dans une position de repos. La figure 1b montre schématiquement le mélangeur 10 sans pale dans une position de fonctionnement. Les figures 1a et 1b sont décrites conjointement.

Le mélangeur 10 comporte un récipient 1 sensiblement cylindrique, d'axe A et de rayon R. Un liquide à mélanger 2 est placé dans le récipient 1. Le liquide à mélanger 2 est caractérisé par une viscosité cinématique v, typiquement exprimée en $m^2/s$. Le liquide à mélanger 2 présente une surface libre 3 à une hauteur H mesurée suivant l'axe A. La surface libre 3 est par définition la surface du liquide à mélanger 2 qui n'est pas en contact avec les parois du récipient 1. La hauteur H peut être définie comme la longueur de l'axe A qui est immergée dans le liquide 2. La hauteur H est mesurée dans la position de repos de la figure 1a. La hauteur H et le rayon R s'expriment typiquement en m.

Dans la position de repos de la figure 1a, l'axe A du récipient 1 est suivant une direction verticale, et le récipient 1 est immobile. On entend par « direction verticale » une direction parallèle à la direction de la force de pesanteur $\vec{g}$.

Dans la position de fonctionnement de la figure 1b, le récipient 1 est incliné et en rotation suivant l'axe A. Le récipient 1 est incliné au moyen d'un organe d'inclinaison, non représenté. L'inclinaison est choisie telle que l'axe A du récipient 1 forme un angle $\alpha$ non nul, inférieur ou égal à 30° par rapport à la direction verticale, L'angle $\alpha$ est préférentiellement inférieur ou égal à 15° et plus préférentiellement inférieur ou égal à 5°. L'angle $\alpha$ peut par exemple être égal à 0,5°, ou à 1°, ou à 5°, ou à 15°. Pour un angle $\alpha$ supérieur à 30°, l'écoulement à la surface libre risque de générer un cisaillement. Le récipient 1 est mis en rotation suivant l'axe A par un organe d'entraînement, non représenté. Le récipient 1 est entraîné dans un mouvement de rotation suivant l'axe A avec une vitesse angulaire de rotation $\Omega$ typiquement exprimée en rad/s.

Le rapport d'aspect H/R de la hauteur H sur le rayon R est choisi de manière à se placer dans une résonance d'un mode propre inertiel du liquide à mélanger 2. Un mode propre inertiel peut être défini comme un mouvement de perturbation global et périodique ou stationnaire du liquide à mélanger 2 placé dans le récipient 1 incliné et en rotation. L'inclinaison de l'axe A par rapport à la verticale permet de forcer le liquide 2 tournant dans un mode propre inertiel. Il existe, pour chaque mode propre inertiel, une pluralité de résonances.

Pour le premier mode propre inertiel du liquide 2 placé dans le récipient 1 incliné et en rotation, le rapport d'aspect H/R est choisi tel que :

$$1,79 \times k \leq \frac{H}{R} \leq 2,19 \times k$$

k étant égal à 1. De manière préférentielle, le rapport d'aspect H/R est choisi sensiblement égal à 1,99. On contribue ainsi à favoriser l'apparition d'une résonance du premier mode propre inertiel.

Pour le deuxième mode propre inertiel du liquide 2 placé dans le récipient 1 incliné et en rotation, le rapport d'aspect H/R est avantageusement choisi tel que :

$$0,86 \times k \leq \frac{H}{R} \leq 1,06 \times k$$

k étant égal à 1. De manière préférentielle, le rapport d'aspect H/R est choisi sensiblement égal à 0,96. On contribue ainsi à favoriser l'apparition d'une résonance du deuxième mode propre inertiel.

Pour le troisième mode propre inertiel du liquide 2 placé dans le récipient 1 incliné et en rotation, le rapport d'aspect H/R est avantageusement choisi tel que :

$$0,56 \times k \leq \frac{H}{R} \leq 0,68 \times k$$

k étant égal à 1. De manière préférentielle, le rapport d'aspect H/R est choisi sensiblement égal à 0,62. On contribue ainsi à favoriser l'apparition d'une résonance du troisième mode propre inertiel.

La vitesse angulaire de rotation Ω du récipient 1 est choisie de manière à observer une résonance instable du mode propre inertiel du liquide 2 placé dans le récipient 1 incliné et en rotation. On entend par « instable » le fait que d'autres mouvements, différents du mouvement du mode propre inertiel, apparaissent au sein du liquide 2 sans contrainte extérieure supplémentaire. Le mélange au sein du liquide 2 est en effet meilleur lorsque la résonance est instable.

Pour le premier mode propre inertiel avec k = 1 et pour une vitesse angulaire de rotation Ω constante, on observe typiquement une résonance instable du mode propre inertiel du liquide 2 placé dans le récipient 1 incliné et en rotation à la condition suivante :

$$\frac{\Omega \times R^2 \times \alpha}{\nu} > 1000$$

avec :

- Ω la vitesse angulaire de rotation exprimée en rad/s,
- R le rayon du récipient 1 exprimé en m,
- α l'angle d'inclinaison entre l'axe A et la direction verticale exprimé en degré °,
- v la viscosité cinématique du liquide à mélanger 2, exprimée en $m^2/s$ dans le cas de liquides miscibles, la viscosité cinématique correspond à la viscosité moyenne des liquides.

Pour le deuxième mode propre inertiel avec k = 1 et pour une vitesse angulaire de rotation Ω constante, on observe typiquement une résonance instable du mode propre inertiel du liquide 2 placé dans le récipient 1 incliné et en rotation à la condition suivante :

$$\frac{\Omega \times R^2 \times \alpha}{\nu} > 15000$$

Pour le troisième mode propre inertiel avec k = 1 et pour une vitesse angulaire de rotation Ω constante, on observe typiquement une résonance instable du mode propre inertiel du liquide 2 placé dans le récipient 1 incliné et en rotation à la condition suivante :

$$\frac{\Omega \times R^2 \times \alpha}{\nu} > 50000$$

Le rayon R, l'angle d'inclinaison α et la viscosité cinématique v étant fixés, les inégalités précédentes permettent d'évaluer la vitesse angulaire de rotation Ω minimum à appliquer au récipient 1 pour créer une résonance instable, dans chacun des premier, deuxième et troisième modes propres inertiels. D'une manière générale, plus la vitesse angulaire de rotation Ω est grande, plus le taux de cisaillement au sein du liquide à mélanger 2 est important et plus la quantité d'énergie nécessaire pour entraîner le récipient 1 en rotation augmente. On recherche donc typiquement la création d'une résonance instable tout en choisissant la plus faible vitesse angulaire de rotation Ω possible.

Alternativement, la vitesse angulaire de rotation Ω peut être variable. Une variation de la vitesse angulaire de rotation Ω inférieure ou égale à 25% au cours d'une révolution complète du récipient 1 est privilégiée.

Dans le présent document, on entend par « récipient cylindrique » un récipient ayant une paroi latérale définie par une droite appelée génératrice, passant par un point variable décrivant une courbe, appelée courbe directrice, et gardant une direction fixe. La courbe directrice est préférentiellement un cercle. Cette forme de récipient est préférée car elle facilite la prévision de l'écoulement d'un liquide placé dans ledit récipient, le récipient étant incliné et en rotation, et la définition du rapport d'aspect H/R permettant l'obtention d'une résonance d'un mode propre inertiel dudit liquide.

Alternativement, la courbe directrice peut être :

- une ellipse, ou
- un polygone convexe inscrit dans un cercle, ou
- un polygone convexe inscrit dans une ellipse.

Toutefois, lorsqu'un liquide est placé dans un récipient incliné et en rotation, le récipient étant de section polygonale, le liquide présente généralement un écoulement comportant des vortex dans les coins, ce qui n'est pas souhaité car un vortex dissipe beaucoup d'énergie.

D'une manière générale, on entend par « rayon du récipient cylindrique d'axe A » la distance la plus petite entre l'axe A du récipient et une paroi latérale du récipient.

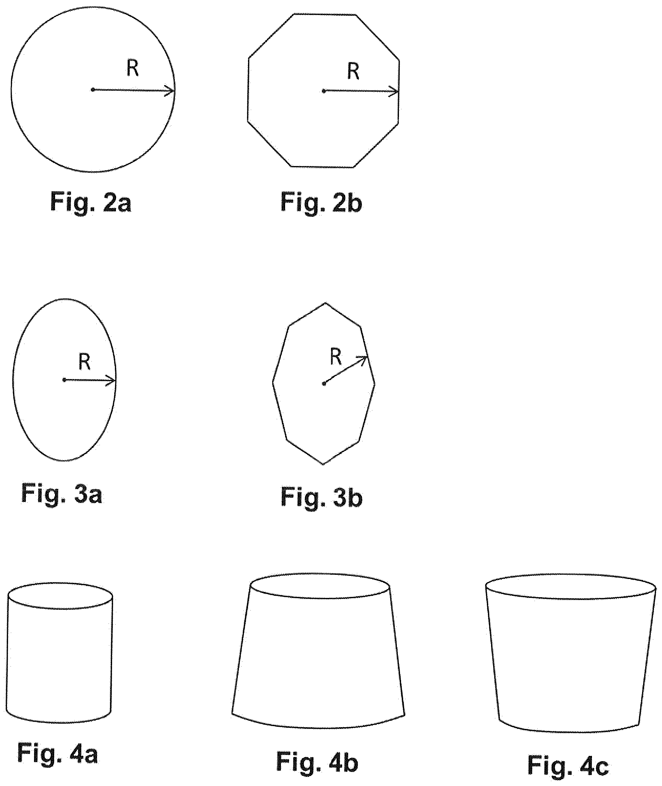

La figure 2a montre une vue en coupe d'un premier récipient cylindrique selon un aspect de l'invention ayant une courbe directrice en forme de cercle. La coupe est réalisée selon un plan perpendiculaire à l'axe de rotation du premier récipient cylindrique.

La figure 2b montre une vue en coupe d'un deuxième récipient cylindrique selon un aspect de l'invention ayant une courbe directrice en forme de polygone convexe à N côtés inscrit dans un cercle. La coupe est réalisée selon un plan perpendiculaire à l'axe de rotation du deuxième récipient cylindrique. Dans l'exemple particulier de la figure 2b, la courbe directrice est un octogone convexe régulier et N = 8. Naturellement, d'autres valeurs de N pourraient être choisies, comme N = 4 ou N = 16. On choisit préférentiellement la plus grande valeur possible de N afin que la courbe directrice tende vers le cercle dans lequel est inscrit le polygone convexe à N côtés.

La figure 3a montre une vue en coupe d'un troisième récipient cylindrique selon un aspect de l'invention ayant une courbe directrice en forme d'ellipse. La coupe est réalisée selon un plan perpendiculaire à l'axe de rotation du troisième récipient cylindrique. On choisit préférentiellement une ellipse dont les deux foyers sont rapprochés l'un de l'autre au maximum afin que la courbe directrice tende vers un cercle.

La figure 3b montre une vue en coupe d'un quatrième récipient cylindrique selon un aspect de l'invention ayant une courbe directrice en forme de polygone convexe à N côtés inscrit dans une ellipse. L'ellipse dans laquelle est inscrit le polygone convexe à N côtés a préférentiellement ses deux foyers rapprochés l'un de l'autre au maximum. La coupe est réalisée selon un plan perpendiculaire à l'axe de rotation du quatrième récipient cylindrique. Dans l'exemple particulier de la figure 3b, la courbe directrice est un octogone convexe inscrit dans une ellipse et N = 8. Naturellement, d'autres valeurs de N pourraient être choisies, comme N = 4 ou N = 16. On choisit préférentiellement la plus grande valeur possible de N afin que la courbe directrice tende vers l'ellipse dans laquelle est inscrit le polygone convexe à N côtés.

On entend par « récipient sensiblement cylindrique » un récipient cylindrique ou tronconique. En effet, les variations de diamètre de la section du récipient ne perturbent pas le fonctionnement du mélangeur dans la mesure où elles restent faibles, c'est-à-dire inférieures ou égales à 20% et préférentiellement inférieures ou égales à 10%. On conçoit que la fabrication du récipient peut conduire à des formes qui ne sont pas parfaitement cylindriques, notamment des formes tronconiques.

La figure 4a montre schématiquement une première vue en perspective d'un récipient sensiblement cylindrique selon un aspect de l'invention : les dimensions de la section du récipient selon un plan perpendiculaire à son axe de rotation sont constantes et le récipient est parfaitement cylindrique.

La figure 4b montre schématiquement une deuxième vue en perspective d'un récipient sensiblement cylindrique selon un aspect de l'invention : les dimensions de la section du récipient selon un plan perpendiculaire à son axe de rotation varient continument ; le récipient est tronconique. Le récipient de la figure 4b présente un fond et une ouverture et le rayon du fond est supérieur au rayon de l'ouverture.

La figure 4c montre schématiquement une troisième vue en perspective d'un récipient sensiblement cylindrique selon un aspect de l'invention : les dimensions de la section du récipient selon un plan perpendiculaire à son axe de rotation varient continument ; le récipient est tronconique. Le récipient de la figure 4c présente un fond et une ouverture et le rayon du fond est inférieur au rayon de l'ouverture.

Les figures 4a à 4c montrent trois exemples de récipients sensiblement cylindriques selon un aspect de l'invention, chaque récipient ayant une courbe directrice circulaire. En accord avec la description réalisée précédemment en lien avec les figures 2a, 2b, 3a et 3b, on comprend que les exemples des figures 4a à 4c peuvent être généralisés à des récipients ayant des courbes directrices en forme d'ellipse, ou en forme de polygone convexe inscrit dans un cercle, ou en forme de polygone convexe inscrit dans une ellipse.

**Revendications**

**1.** Mélangeur sans pale pour le mélange d'un liquide comportant :

- un récipient (1) cylindrique ou tronconique d'axe A et de rayon R, le rayon R étant la plus petite distance entre l'axe A du récipient et une paroi latérale du récipient, le liquide à mélanger (2) étant placé dans le récipient et présentant une surface libre (3) à une hauteur H mesurée suivant l'axe A, dans une position de repos verticale, le récipient (1) cylindrique étant un récipient ayant une paroi latérale définie par une droite, passant par un point variable décrivant une courbe, appelée courbe directrice, et gardant une direction fixe; la courbe directrice étant un cercle, ou une ellipse, ou un polygone convexe inscrit dans un cercle, ou un polygone convexe inscrit dans une ellipse; le récipient (1) tronconique ayant des variations de diamètre de section inférieures ou égales à 20% ;
- un organe d'inclinaison du récipient (1) de manière que l'axe A forme un angle $\alpha$ non nul choisi inférieur ou égal à 30° par rapport à la direction verticale;

- un organe adapté pour l''entraînement du récipient (1) selon un mouvement de rotation suivant l'axe A et avec une vitesse angulaire de rotation $\Omega$ ;

le rapport d'aspect H/R de la hauteur H sur le rayon R et la vitesse angulaire de rotation $\Omega$ sont choisis de manière à observer une résonance instable d'un mode propre inertiel du liquide à mélanger lorsque le récipient est incliné et en rotation, **caractérisé en ce que** la hauteur H, le rayon R et la vitesse angulaire de rotation $\Omega$ sont choisis tel que :

$$1,79 \leq \frac{H}{R} \leq 2,19 \text{ et } \frac{\Omega \times R^2 \times \alpha}{\nu} > 1000 \text{ ;}$$

$$0,86 \leq \frac{H}{R} \leq 1,06 \text{ et } \frac{\Omega \times R^2 \times \alpha}{\nu} > 15000 \text{ ;}$$

ou

$$0,56 \leq \frac{H}{R} \leq 0,68 \text{ et } \frac{\Omega \times R^2 \times \alpha}{\nu} > 50000;$$

avec $\Omega$ la vitesse angulaire de rotation exprimée en rad/s, R le rayon du récipient exprimé en m, H la hauteur du liquide en m ; $\alpha$ l'angle d'inclinaison entre l'axe A et la direction verticale exprimée en degré ° et v la viscosité cinématique du liquide à mélanger (2) exprimée en m$^2$/s.

2. Mélangeur selon la revendication 1, pour lequel un premier mode propre inertiel du liquide à mélanger est excité.

3. Mélangeur selon la revendication 1, pour lequel un deuxième mode propre inertiel du liquide à mélanger est excité.

4. Mélangeur selon la revendication 1, pour lequel un troisième mode propre inertiel du liquide à mélanger est excité.

5. Mélangeur sans pale selon l'une quelconque des revendications précédentes pour lequel :

   - la vitesse angulaire de rotation $\Omega$ est variable, et
   - la variation de la vitesse angulaire de rotation $\Omega$ est inférieure ou égale à 25% au cours d'une révolution complète du récipient.

6. Procédé de mélange d'un liquide (2) au moyen d'un mélangeur sans pale comportant un récipient (1) cylindrique ou tronconique d'axe A et de rayon R, le récipient (1) cylindrique étant un récipient ayant une paroi latérale définie par une droite, passant par un point variable décrivant une courbe, appelée courbe directrice, et gardant une direction fixe; la courbe directrice étant un cercle, ou une ellipse, ou un polygone convexe inscrit dans un cercle, ou un polygone convexe inscrit dans une ellipse, le récipient (1) tronconique ayant des variations de diamètre de section inférieures ou égales à 20%, le rayon R étant la distance la plus petite entre l'axe A du récipient et une paroi latérale du récipient, un organe d'inclinaison du récipient (1) de manière que l'axe A forme un angle $\alpha$ non nul, choisi inférieur ou égal à 30° par rapport à la direction verticale ;et un organe d'entraînement du récipient (1), le procédé comportant les étapes suivantes :

   a) placer le liquide (2) dans le récipient (1) de manière qu'il présente une surface libre (3) à une hauteur H mesurée suivant l'axe A, dans une position de repos verticale ;
   b) appliquer un mouvement de rotation au récipient (1) suivant l'axe A et avec une vitesse angulaire de rotation $\Omega$, le rapport d'aspect H/R de la hauteur H sur le rayon R et la vitesse angulaire de rotation $\Omega$ étant choisis de manière à observer une résonance instable d'un mode propre inertiel du liquide (2),

**caractérisé en ce que**

-

$$1{,}79 \leq \frac{H}{R} \leq 2{,}19 \text{ et } \frac{\Omega \times R^2 \times \alpha}{\nu} > 1000 \; ;$$

-

$$0{,}86 \leq \frac{H}{R} \leq 1{,}06 \text{ et } \frac{\Omega \times R^2 \times \alpha}{\nu} > 15000 \; ;$$

ou

-

$$0{,}56 \leq \frac{H}{R} \leq 0{,}68 \text{ et } \frac{\Omega \times R^2 \times \alpha}{\nu} > 50000;$$

avec $\Omega$ la vitesse angulaire de rotation exprimée en rad/s, R le rayon du récipient exprimé en m, H la hauteur du liquide en m mesurée suivant l'axe A ; $\alpha$ l'angle d'inclinaison entre l'axe A et la direction verticale exprimé en degré ° et v la viscosité cinématique du liquide à mélanger (2) exprimée en m²/s.

7. Procédé selon la revendication 6 pour lequel le mode propre inertiel du liquide (2) est le premier, second ou troisième mode.


**Patentansprüche**

1. Mischer ohne Flügel zum Mischen einer Flüssigkeit, bestehend aus:

- einem zylindrischen oder kegelstumpfförmigen Behälter (1) mit einer Achse A und einem Radius R, wobei der Radius R der kleinste Abstand zwischen der Achse A des Behälters und einer Seitenwand des Behälters ist, wobei die zu mischende Flüssigkeit (2) in den Behälter eingefüllt ist und eine freie Oberfläche (3) in einer Höhe H aufweist, die entlang der Achse A in einer vertikalen Ruheposition gemessen wird, wobei der zylindrische Behälter (1) ein Behälter mit einer Seitenwand ist, die durch eine Gerade definiert ist, die durch einen variablen Punkt verläuft, der eine Kurve beschreibt, die als Leitkurve bezeichnet wird, und eine feste Richtung beibehält; wobei die Leitkurve ein Kreis oder eine Ellipse oder ein in einen Kreis eingeschriebenes konvexes Vieleck oder ein in eine Ellipse eingeschriebenes konvexes Vieleck ist; wobei der kegelstumpfförmige Behälter (1) Querschnittsdurchmesseränderungen von weniger als oder gleich 20 % aufweist;
- einem Element zum Neigen des Behälters (1), so dass die Achse A einen Winkel $\alpha$ ungleich Null bildet, der kleiner oder gleich 30° zur vertikalen Richtung ist;
- einem Element, das zum Antreiben des Behälters (1) in einer Drehbewegung entlang der Achse A und mit einer Drehwinkelgeschwindigkeit $\Omega$ geeignet ist;
das Seitenverhältnis H/R der Höhe H zum Radius R und die Drehwinkelgeschwindigkeit $\Omega$ sind so gewählt, dass eine instabile Resonanz eines eigenen Trägheitsmodus der zu mischenden Flüssigkeit beobachtet wird, wenn der Behälter geneigt ist und sich dreht, **dadurch gekennzeichnet, dass** die Höhe H, der Radius R und die Drehwinkelgeschwindigkeit $\Omega$ so gewählt sind, dass:

-

$$1{,}79 \leq \frac{H}{R} \leq 2{,}19 \text{ und } \frac{\Omega \times R^2 \times \alpha}{\upsilon} > 1000 \; ;$$

-

$$0{,}86 \leq \frac{H}{R} \leq 1{,}06 \text{ und } \frac{\Omega \times R^2 \times \alpha}{\upsilon} > 15000 \; ;$$

oder
-

$$0{,}56 \leq \frac{H}{R} \leq 0{,}68 \text{ und } \frac{\Omega \times R^2 \times \alpha}{v} > 50000 \ ;$$

mit der Drehwinkelgeschwindigkeit $\Omega$, ausgedrückt in rad/s, R dem Radius des Behälters, ausgedrückt in m, H der Höhe der Flüssigkeit in m; $\alpha$ dem Neigungswinkel zwischen der Achse A und der vertikalen Richtung, ausgedrückt in Grad °, und v der kinematischen Viskosität der zu mischenden Flüssigkeit (2), ausgedrückt in m$^2$/s.

2. Mischer nach Anspruch 1, bei dem ein erster eigener Trägheitsmodus der zu mischenden Flüssigkeit angeregt wird.

3. Mischer nach Anspruch 1, bei dem ein zweiter eigener Trägheitsmodus der zu mischenden Flüssigkeit angeregt wird.

4. Mischer nach Anspruch 1, bei dem ein dritter eigener Trägheitsmodus der zu mischenden Flüssigkeit angeregt wird.

5. Mischer ohne Flügel gemäß einem der vorstehenden Ansprüche, bei dem:

   - die Drehwinkelgeschwindigkeit $\Omega$ variabel ist und
   - die Änderung der Drehwinkelgeschwindigkeit $\Omega$ während einer vollständigen Umdrehung des Behälters kleiner oder gleich 25 % ist.

6. Verfahren zum Mischen einer Flüssigkeit (2) mittels eines Mischers ohne Flügel mit einem zylindrischen oder kegelstumpfförmigen Behälter (1) mit einer Achse A und einem Radius R, wobei der zylindrische Behälter (1) ein Behälter mit einer Seitenwand ist, die durch eine Gerade definiert ist, die durch einen variablen Punkt verläuft, der eine Kurve beschreibt, die als Leitkurve bezeichnet wird, und eine feste Richtung beibehält; wobei die Leitkurve ein Kreis oder eine Ellipse oder ein in einen Kreis eingeschriebenes konvexes Vieleck oder ein in eine Ellipse eingeschriebenes konvexes Vieleck ist, wobei der kegelstumpfförmige Behälter (1) Querschnittsdurchmesseränderungen von weniger als oder gleich 20 % aufweist, wobei der Radius R der kleinste Abstand zwischen der Achse A des Behälters und einer Seitenwand des Behälters ist, ein Element zum Neigen des Behälters (1) derart, dass die Achse A einen Winkel $\alpha$ ungleich Null bildet, der kleiner oder gleich 30° zur vertikalen Richtung gewählt ist; und ein Element zum Antreiben des Behälters (1), wobei das Verfahren die folgenden Schritte umfasst:

   a) Einfüllen der Flüssigkeit (2) in den Behälter (1) derart, dass sie in einer vertikalen Ruheposition eine freie Oberfläche (3) in einer Höhe H aufweist, die entlang der Achse A gemessen wird;
   b) Ausüben einer Drehbewegung auf den Behälter (1) entlang der Achse A und mit einer Drehwinkelgeschwindigkeit $\Omega$, wobei das Seitenverhältnis H/R der Höhe H zum Radius R und die Drehwinkelgeschwindigkeit $\Omega$ so gewählt sind, dass eine instabile Resonanz eines eigenen Trägheitsmodus der Flüssigkeit (2) beobachtet wird,

**dadurch gekennzeichnet, dass**

   -

$$1{,}79 \leq \frac{H}{R} \leq 2{,}19 \text{ und } \frac{\Omega \times R^2 \times \alpha}{v} > 1000 \ ;$$

   -

$$0{,}86 \leq \frac{H}{R} \leq 1{,}06 \text{ und } \frac{\Omega \times R^2 \times \alpha}{v} > 15000 \ ;$$

   oder
   -

$$0{,}56 \leq \frac{H}{R} \leq 0{,}68 \text{ und } \frac{\Omega \times R^2 \times \alpha}{v} > 50000 \ ;$$

mit $\Omega$ der Drehwinkelgeschwindigkeit in rad/s, R dem Radius des Behälters in m, H der Höhe der Flüssigkeit in m, gemessen entlang der Achse A; $\alpha$ dem Neigungswinkel zwischen der Achse A und der vertikalen Richtung in Grad ° und v der kinematischen Viskosität der zu mischenden Flüssigkeit (2) in m²/s.

7. Verfahren nach Anspruch 6, bei dem der eigene Trägheitsmodus der Flüssigkeit (2) der erste, zweite oder dritte Modus ist.

**Claims**

1. Bladeless mixer to mix a liquid, comprising:

   - a cylindrical or tapered container (1) of axis A and radius R, where radius R is the smallest distance between axis A of the container and a side wall of the container, where the liquid to be mixed (2) is placed in the container, and where it has a free surface (3) of height H measured along axis A;
   - a device to tilt the container (1) such that axis A forms a non-zero angle $\alpha$, chosen to be less than or equal to 30° relative to vertical;
   - a device to drive the container (1) with a rotational motion around axis A, at an angular speed of rotation $\Omega$;

   aspect ratio H/R of height H over radius R and angular speed of rotation $\Omega$ are chosen so as to observe an unstable resonance of an inertial eigenmode of the liquid to be mixed when the container is tilted and rotating **characterised in that** height H over radius R and angular speed of rotation $\Omega$ are chosen such that:

   -

$$1{,}79 \leq \frac{H}{R} \leq 2{,}19 \text{ and } \frac{\Omega \times R^2 \times \alpha}{\nu} > 1000 \; ;$$

   -

$$0{,}86 \leq \frac{H}{R} \leq 1{,}06 \text{ and } \frac{\Omega \times R^2 \times \alpha}{\nu} > 15000 ;$$

   or
   -

$$0{,}56 \leq \frac{H}{R} \leq 0{,}68 \text{ and } \frac{\Omega \times R^2 \times \alpha}{\nu} > 50000 ;$$

   where $\Omega$ is the angular speed of rotation expressed in rad/s, R is the radius of the container expressed in m, H the height of the liquid in m measured along axis A; $\alpha$ the angle of tilt between axis A and vertical, expressed in degrees °, and v the kinematic viscosity of the liquid to be mixed (2), expressed in m²/s.

2. Mixer according to claim 1, for which the first inertial eigenmode of the liquid to be mixed is excited.

3. Mixer according to claim 1, for which the second inertial eigenmode of the liquid to be mixed is excited.

4. Mixer according to claim 1, for which the third inertial eigenmode of the liquid to be mixed is excited.

5. Bladeless mixer according to any of the previous claims, for which:

   - angular speed of rotation $\Omega$ is variable, and
   - the variation of angular speed of rotation $\Omega$ is less than or equal to 25% during a complete revolution of container (1).

6. Method of mixing a liquid (2) by means of a bladeless mixer containing a cylindrical or tapered container (1) of axis A and radius R, where R is the smallest distance between axis A of the container and a side wall of the container, a device to tilt the container (1) such that axis A forms a non-zero angle $\alpha$, chosen such that it is less than or equal to 30° relative to vertical; and a device to drive the container (1), where the method includes the following steps:

a) placing the liquid (2) in the container (1) such that it has a free surface (3) at a height H measured along axis A;
b) applying a rotational motion to the container (1) around axis A, with an angular speed of rotation $\Omega$, where aspect ratio H/R of height H over radius R and angular rotational speed $\Omega$ are chosen so as to observe an unstable resonance of an inertial eigenmode of the liquid (2)

**characterised in that**:

-

$$1,79 \leq H/R \leq 2,19 \text{ and } (\Omega \times R\text{^}2 \times \alpha)/ v > 1000 ;$$

-

$$0,86 \leq H/R \leq 1,06 \text{ and } (\Omega \times R\text{^}2 \times \alpha)/ v > 15000;$$

or
-

$$0,56 \leq H/R \leq 0,68 \text{ and } (\Omega \times R\text{^}2 \times \alpha)/ v > 50000;$$

where $\Omega$ is the angular speed of rotation expressed in rad/s, R is the radius of the container expressed in m, H the height of the liquid in m measured along axis A; $\alpha$ the angle of tilt between axis A and vertical, expressed in degrees °, and v the kinematic viscosity of the liquid to be mixed (2), expressed in m$^2$/s.

7. Method according to claim 6 for which the inertial eigenmode of the liquid (2) is the first, second or third mode.

**Fig. 1a**

**Fig. 1b**

**Fig. 1c**

Fig. 2a

Fig. 2b

Fig. 3a

Fig. 3b

Fig. 4a

Fig. 4b

Fig. 4c

## RÉFÉRENCES CITÉES DANS LA DESCRIPTION

**Documents brevets cités dans la description**

- FR 1362354 A

- EP 2893973 A1